# EUROPEAN PATENT SPECIFICATION

(11) **EP 1 251 884 B1**
(45) Date of publication and mention of the grant of the patent: **05.07.2006**
(21) Application number: 00930789.3
(22) Date of filing: 18.05.2000
(51) Int. Cl.: A61L 9/014, A61L 9/12

(54) **AIR FILTERING DEVICE**
LUFTFILTEREINRICHTUNG
DISPOSITIF DE FILTRATION D'AIR

(30) Priority: 04.02.2000 WO PCT/US00/03010
(43) Date of publication of application: 30.10.2002
(73) Proprietor: THE PROCTER & GAMBLE COMPANY, Cincinnati, Ohio 45202 (US)
(72) Inventor: KVIETOK, Frank, Andrej, Cincinnati, OH 45208 (US); LIU, Zaiyou, West Chester, OH 45069 (US); PALJIEG, Steve, Mason, OH 45040 (US); REDMON, Sonia, Cincinnati, OH 45215 (US); SPADINI, Alessandro, Cincinnati, OH 45215 (US)
(74) Representative: Peet, Jillian Wendy
(86) International application number: PCT/US2000/013531
(87) International publication number: WO 2001/056620

(56) References cited:
- EP-A- 0 311 454
- DE-A- 3 002 409
- DE-A- 3 640 953
- US-A- 3 049 399
- US-A- 3 972 678
- US-A- 4 235 750
- US-A- 5 772 738
- DATABASE WPI Section Ch, Week 199918 Derwent Publications Ltd., London, GB; Class D22, AN 1999-208546 XP002148971 & JP 11 047256 A (KAWAI MUSICAL INSTR MFG CO), 23 February 1999 (1999-02-23)
- DATABASE WPI Section Ch, Week 199151 Derwent Publications Ltd., London, GB; Class A88, AN 1991-373442 XP002148972 & JP 03 251253 A (DAISO CO LTD), 8 November 1991 (1991-11-08)
- DATABASE WPI Section Ch, Week 199524 Derwent Publications Ltd., London, GB; Class D14, AN 1995-181605 XP002148973 & JP 06 288672 A (GOLDSTAR CO LTD), 18 October 1994 (1994-10-18) -& JP 06 288672 A (GOLDSTAR CO LTD) 18 October 1994 (1994-10-18)

## Description

### Field of the invention

The present invention relates to air filtering devices for removing malodor and ethylene from the air. Such devices are useful for example for storing and preserving food in closed compartments such as refrigerators.

### Background

Nowadays, refrigerators have become a common appliance in virtually every household and typically are used for storage and preservation of food in particular of fresh food such as fruits, vegetables, dairy products, and the like. It is desirable to keep the food items fresh as long as possible in the refrigerator.

It is a well known problem that many food items tend to release malodors into the air which are then captured in the limited air space in a refrigerator. Not only are these malodors unpleasant and offensive to the user of the refrigerator, they can also have a negative impact on the quality of other foods in the refrigerator. For example, it is known that some foods emit strong odors (e.g. fish, boiled eggs, onions, etc.) and that these odors can transfer to other nearby foods and hurt the taste and freshness of those foods. A common example is transfer of odors into an open container of orange juice or of milk resulting in a noticeable degradation in their taste. It is also well known that malodors from some vegetables (onions, garlic) can transfer to other foods stored within a vegetable drawer. This problem is aggravated when the vegetable drawer is sealed such that there is very little air exchange with the larger compartment of the refrigerator (herein referred to as the "fresh food compartment") and when vegetables have been cut or are stored without any outer wrapping. This problem of odor transfer is particularly acute in the case of ice cubes where odors from the fresh food compartment of the refrigerator can be transferred to the ice in the freezer compartment of the refrigerator. This is especially true in the case of refrigerators in which there is air exchange between the fresh food and freezer compartments, and especially in the case of refrigerators with built-in ice-makers.

In addition to malodors, however, fruits and vegetables are known to produce ethylene as a part of their normal ripening process. Unfortunately this ethylene also contributes to the degradation of ethylene sensitive foods, primarily vegetables like broccoli, lettuce, green peppers, etc. Ethylene-producing and ethylene-sensitive fruits and vegetables are well known.

U.S. patent No. 5,403,548 discloses an activated carbon absorbent to be used for example in refrigerators, shoe boxes, closets, toilets, cars, cupboard, or the like. The activated carbon absorbent is applied in a gas treating apparatus comprising an air inlet, and air outlet, a cylinder housing the activated carbon honeycomb, and a fan aspiring malodor through the air inlet. Change of battery and withdrawal of the cylinder housing the activated carbon are achieved by dismounting the cover of the apparatus. The gas treating apparatus may further comprise an action means for alerting the user to the event that the useful life of the activated carbon adsorbent has run out. This gas treating apparatus has the disadvantage that it is not capable of removing ethylene from the air.

It is therefore an object of the present invention to provide an air filtering device which overcomes the disadvantages of the prior art devices.

It is a further object of the present invention to provide an air filtering device which is capable of removing both malodors and ethylene from the air.

### Summary of the invention

The present invention provides an air filtering device for filtering air in confined spaces such as refrigerators, food storage closets, pantries, coolers and the like, comprising a first filtering member, a second filtering member, and an air flow conduit defining an air flow path through the device from an air inlet to an air outlet. The first filtering member and the second filtering member are positioned to interact with at least a portion of the molecules flowing along the air flow path or being adsorbed in one of the filter members . The air filtering device of the present invention is characterized in that the first filtering member is capable of removing malodors from the air and comprises activated carbon supported on a support member, and the second filtering member is capable of oxidizing ethylene and comprises potassium permanganate supported on a zeolite or on alumina and wherein less than 30% of the macroscopic surface of the potassium permanganate is immediately exposed to air and at least 30% of the macroscopic surface of the potassium permanganate is in contact with activated carbon.

### Brief description of the figures

Fig. 1 is a schematic view showing one example of a filter member according to the present invention.
Fig. 2 is a schematic cross-section of the filter medium shown in the example of Fig. 1.
Fig. 3 is a schematic assembly view of an example of a device of the current invention.
Fig. 4 is a schematic drawing of an alternate example of the device of the present invention.
Fig. 5 is a schematic drawing of an alternate example of the device of the present invention.

### Detailed description of the invention

The device of the present invention is intended to filter air in confined spaces such as for example in refrigerators, food storage closets, pantries, coolers, and the like. The simultaneous removal of malodors and ethylene has been found to be particularly beneficial for keeping food items such as fruits and vegetables fresh for an extended period of time. In addition, transfer of malodors or taste from one food item to another one is prohibited.

Filtering of the air to remove malodors in the device the present invention may be achieved by adsorbing the molecules constituting a malodor onto a surface of a filter member. The term "adsorption" is well defined in the art and refers to the adherence of molecules to surfaces which effectively reduces the mobility of these molecules to the two dimensions of the surface. Those molecules remaining in the air will then diffuse so that further molecules come into contact with the surface and subsequently will be adsorbed. Consequently, most of the malodor molecules will travel into the proximity of one of the surfaces at some point in time so that finally most of the malodor will be removed from the air.

A suitable filter member for removing malodors comprises activated carbon for the adsorption. Activated carbon is known to be a very effective filter medium due to its high specific surface area. Whilst activated carbon is very effective as such, the filter member of the present invention may further comprise agents supported on the activated carbon to specifically attack certain malodors such as those comprising S atoms or N atoms. Preferably, the filter members of the present invention comprise at least 2 grams, more preferably at least 5 grams, and most preferably at least 10 grams of activated carbon. Preferably, the filter members of the present invention comprise less than 100 grams, more preferably less than 50 grams, yet more preferably less than 40 grams, and most preferably less than 30 grams of activated carbon.

A wide variety of activated carbon based filter media is known in the art. The carbon for the media could come from any source. Coconut shells are a common example of a source of activated carbon.

The filter member of the present invention comprise a support for the activated carbon for example in the form of a foam, a nonwoven web or a woven web, or an open-pore or reticulate structure, such as a foam or a mesh.

Although activated carbon is used in many odor control applications in the form of granules and pellets, these forms have disadvantages for the present invention. First, these particle forms, because of their low ratio of surface area to mass, do not facilitate rapid adsorption of odors from a confined space. Second they introduce a high pressure drop which further impedes adsorption and their effectiveness at removing odors. In conjunction with an air moving means, the pressure drops across these beds of granules or pellets require a relatively high operating power to achieve the air flow rates needed to get fast and effective odor removal. This is a critical disadvantage for a product designed for use in confined spaces, as the addition of more and more power leads to bigger, more costly, and noisier devices for these applications.

While the adsorption rates can be increased by using powder forms of activated carbon with much smaller particle sizes than granular activated carbon, the powder forms are difficult to handle and further increase the pressure drop issue described above.

It has been found that this pressure drop problem can be overcome through the use of an activated carbon filter member wherein the activated carbon is supported on an support structure, such as open-pore reticulate structure, a foam or mesh which provides flow-through channels for air contacting with activated carbon particles supported on and within the support structure.

The use of such supported forms of activated carbon allows one to obtain high thermodynamic capacity for adsorbing odors while at the same time enabling rapid adsorption due to the low pressure drop and high degree of contacting between the individual activated carbon particles and the air. In combination with an air moving means, this preferred filter structure allows very rapid removal of odors in confined spaces even with a device having a relative small power supply such as a single battery and having a relatively small size compared to the spaces in which it is used.

Suitable forms of supported activated carbon structures preferably have an overall member density of between 0.01 to .40 g/cc, more preferably between 0.05 to 0.35 g/cc, even more preferably between 0.10 to 0.30 g/cc, and most preferably between 0.15 and 0.25 g/cc.

Satisfactory performance is achieved when the activated carbon particles in the structure have a specific surface area of about 600 to 2000 m2/g and when the activated carbon density within the filter member is between 0.01 to 0.32 g/cc, more preferably between 0.02 to 0.30 g/cc, even more preferably between 0.08 to 0.25 g/cc, and most preferably between 0.10 to 0.20 g/cc.

Suitable forms of supported activated carbon are the reticulated polyurethane foam products which are commercially available from Helsa-Werke, Helmut Sandler CmbH & Co KG, Germany, under the designations Helsa-tech 8126, 8139, 5600, and 5615.

Removing of ethylene from the air in the device the present invention is achieved by means of the oxidation agent potassium permanganate in a solid form. The oxidation agent is supported on a solid particle, i.e. a zeolite or alumina. The oxidation agent or the solid particle material supporting the oxidation agent may then be supported on a foam or layered in or between nonwoven web materials, woven web materials, or the like. When ethylene comes into contact with oxidation agent, it is oxidized to carbon dioxide and water. Preferably, the filter member of the device of the present invention contains potassium permanganate on alumina and contains at least 0.01 grams of potassium permanganate. Of course, the amount of potassium permanganate needed to achieve optimum performance depends on the circumstances of the specific use and hence may have to and can be adopted by the skilled person.

A suitable oxidation agent member for the present invention comprising potassium permanganate is commercially available from Purafil Inc. of Doravill, GA, USA, under the designation "Purafil P800".

The filter member of the present invention comprises an air inlet, and air outlet, and air flow path through the filter member from the air inlet to the air outlet. The filter medium is disposed in the filter member of the present invention such that it comes into contact with the air flowing along the air flow path. The filter medium may be arranged as a flow by filter or as a flow through filter.

Suitable filter members for the method of the present invention include passive filter members and forced air filter members. The term "passive filter member" as used herein refers to those filter members which only rely on air convection and on diffusion to bring malodors within reach of the filter media in the member. The term "forced air filter member" as used herein refers to those filter members which can be attached to a forced air moving member which draws air into the device through a filter member containing a filter media and increases air flow through the filter media above that achieved through normal air convection in the confined space. As used within this disclosure, a forced air filter member consists of a filter member and an air moving member. A filter member suitable for the forced air filter members may be a passive filter member according to the above definition.

The deodorization of the air and the removal of ethylene in the device of the present invention is enhanced by increasing the air flow through the filter member by means of an air moving member. Preferably, the air moving means moves at least 10 ml of air per second through the air inlet into the device, more preferably at least 100 ml/s, most preferably at least 300ml/s.

Of course, the optimum rate of air flow strongly depends on the particular circumstances of the specific use. Hence, it may necessary for the skilled person to adjust the air flow rate accordingly. There are known in the art a wide variety of suitable air moving members such as for example fans and blowers. A particularly suitable fan is a centrifugal fan. A suitable member for driving the fan is a small motor, for example a DC motor available from MABUCHI MOTOR CO., LTD., Japan, under the designation of RF-330TK. The air moving members of the present invention may be powered electrically. Many electrical power sources could be imagined including domestic AC electrical power or power from a static power supply. Alternatively and preferably electrical power may be supplied by means of a battery, preferably a dry alkaline cell battery, or a rechargeable battery. Any replaceable power supply preferably is designed to last at least one month, more preferably at least two months, yet more preferably at least three months, most preferably at least four months:

The device of the present invention may comprise a switch member periodically switching the air moving member on and off to conserve energy. The switch member may coupled to a sensor member which detects the need to operate the air moving member such as by determining the odor or ethylene concentration.

The filter medium is constructed such that surface area of potassium permanganate which is immediately exposed to the air in the filter medium is minimized. The term "immediately exposed to air- as used herein refers to configurations in which the surface of the potassium permanganate is not in contact with other surfaces such as activated carbon, carrier structures, the housing of the filter, and the like. Less than 30% of the macroscopic surface area of the potassium permanganate, preferably less than 20% of the macroscopic surface area of the potassium permanganate more preferably less than 10% of the macroscopic surface area of the potassium permanganate are immediately exposed to air.

The term "macroscopic surface area" as used herein refers to the surface area of the potassium permanganate which is accessible to a probe having a square head of 1cm² surface area, i.e. by disregarding corrugations having an extension of less than 1cm. Consequently, the macroscopic surface area of potassium supported on a web material would for example be the surface area of the two major surfaces of the web material.

To further reduce immediate air contact, it is preferred that the air flow is directed to flow by the potassium permanganate. If the potassium permanganate is arranged in an essentially two dimensional configuration such as on a web material, the air flow should be directed to not flow through the web.

Preferably, the potassium permanganate on its carrier is arranged such that it is in contact with the surface of the activated carbon such that ethylene molecules adsorbed on the carbon surface may migrate to the permanganate and ultimately be oxidized by the permanganate. At least 30% of the macroscopic surface area of the potassium permanganate, preferably at least 40% of the macroscopic surface area of the potassium permanganate, and more preferably at least 50% of the macroscopic surface area of the potassium permanganate are in contact with activated carbon.

Without wishing to be bound by this theory, it is believed that by minimizing immediate exposure of the permanganate to air and by bringing the permanganate into contact with the activated carbon, a surprising increase in ethylene removal performance is achieved. The permanganate by itself appears to be much less effective in removing much ethylene at all in passive mode. The oxidative reaction must be catalyzed by activated carbon in order to occur. Exposing as much activated carbon surface as possible to the air not only optimizes malodor control, but it accelerates ethylene removal by more rapidly creating a high ethylene concentration region around the permanganate fabric. Since activated carbon cannot 'hold' ethylene very effectively within its matrix, it releases it slowly creating ideal conditions for the permanganate fabric (which is very poor kinetically by itself) to destroy it. Hence, placing the two elements as close to one another as possible is best for ethylene removal. One can imagine that in a situation in which ethylene is adsorbing and de-adsorbing within the activated carbon matrix all the time, the oxidation of ethylene at the fringes of the activated carbon foam creates an ethylene concentration gradient. This creates a radial driving force that evacuates the activated carbon of ethylene.

In one suitable embodiment, the filter medium is constructed such that the ethylene-removing material mentioned previously is wrapped radially around a cylindrical or semi-spherical foam/carbon filter. In this configuration, the permanganate fabric is not immediately exposed to the air, and the permanganate is in contact with activated carbon on one side and the filter enclosure on the other. In addition, this orientation is ideal for minimizing processing cost: The permanganate need not be embedded into the activated carbon and very little work has to be done to contain it within the filter. No aesthetic aides are needed as the permanganate is hidden from the filter inlet and outlet. No fabric is wasted in processing as it is cut into rectangular strips rather than more complicated shapes such as disks. The lowest pressure drop possible is achieved (active mode) as only one of the filtering elements is exposed to direct airflow; this minimizes power draw and maximizes battery life. The best malodor control is achieved as the largest possible surface area of activated carbon is exposed to the air.

Surprisingly, it has been found that this configuration maximizes ethylene removal rates and ethylene removal capacity in both active and passive mode. In active mode, the filter is superior to conventional ethylene removal devices in terms of ethylene removal rates while still being constructed without the use of more complicated technologies such as spray coating or embedding. Additionally, this configuration seems to minimize oxidation of other species thereby maximizing the oxidative capacity of permanganate and preventing the creation of unfavorable intermediates such as acetaldehyde, frequently mentioned in literature as a drawback of permanganate oxidation systems.

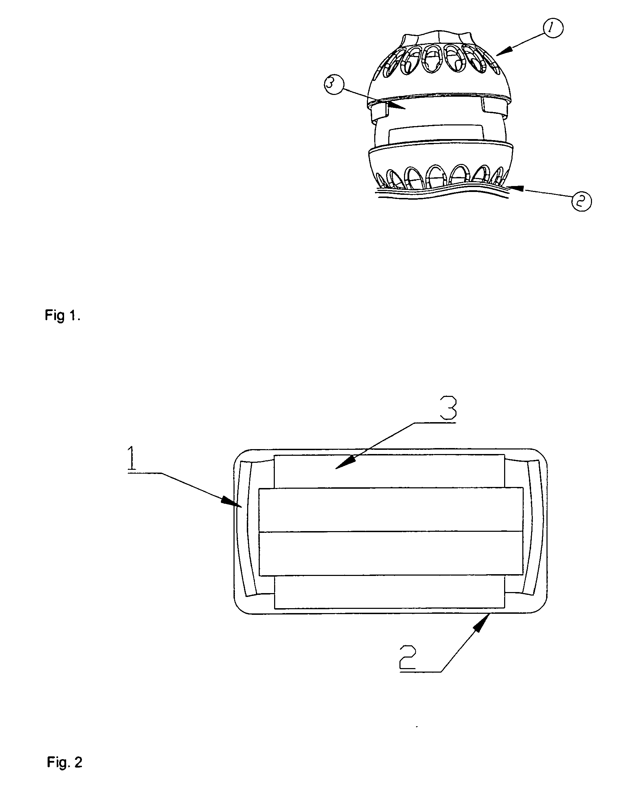

Fig. 1 is a schematic view showing one example of a filter member. The filter member of the present invention comprises an air inlet (1), an air outlet (2) , and air flow path through the filter member (3) from the air inlet to the air outlet. The filter medium is disposed in the filter member of the present invention such that it comes into contact with the air flowing along the air flow path. Preferably, the filter medium contains a carbon filter that is in the general shape of a cylinder, more preferably in the shape of several disks of various diameters and thicknesses such that when the disks are placed atop one another they approach the shape of a sphere or a portion thereof.

Fig 2. Is a schematic cross-section of the filter medium shown in the example of Fig. 1. The filter medium consists of several disks of activated carbon supported by foam (3). The disks are placed atop one another. Around this stack is wrapped a piece of nonwoven material (1) supporting potassium permanganate on alumina. To prevent spillage of carbon particles, this entire assembly is contained in a piece of woven nylon (2) which is stretched tight. The nylon is stretched to a point such that the average pore size of the nylon is less than 0.5 mm².

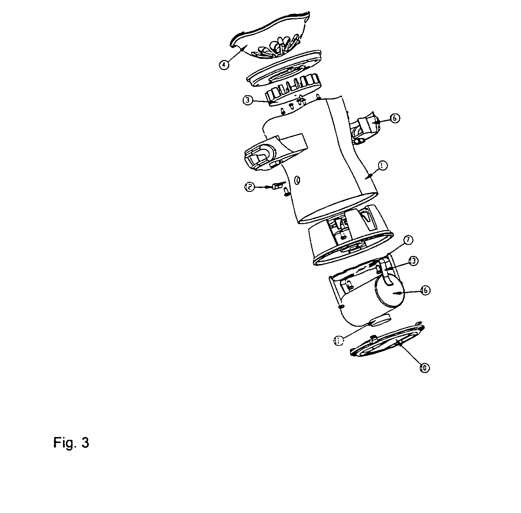

Fig 3. is a schematic assembly view of an example of a device of the current invention. The device comprises a main housing (1) into which two air outlet fixtures (6) are installed. The main housing is sized to contain all of the elements described below. In the preferred mode of this example the filter member of Fig 1 is placed on top of the air inlet (4) such that air is drawn through the filter member via a suction force. A particularly suitable fan to deliver this suction force is a centrifugal fan (3). The centrifugal fan is contained in a small chamber which helps to maximize the air flow, allowing air to be drawn in near the center of the impeller and expelled perpendicular to the entry direction and through the air outlets of the device. The air outlet fixtures are positioned in the main housing such that the rotation of the fan is visible through these outlets. This allows a means of confirming the action of the fan. Preferably, the air moving member moves at least 100 ml of air per second through the air inlet into the device, more preferably at least 200 ml/s most preferably at least 300 ml/s. A suitable member for driving the fan is a small motor (18), for example a DC motor available from MABUCHI Motor Co., LTD., Japan, under the designation of RF-330TK.

The motor is controlled by a circuit board (7). In this example, power for the motor is supplied by means of a battery(16), preferably a 1.5 V dry alkaline cell battery, or a rechargeable battery, which is connected to the circuit board by two battery contacts (13). The battery is held into the device by a battery door (10) onto which is adhered a small piece of foam (11) which helps to maintain a tight fit of the battery into the device. The circuit board also contains circuitry controlling an LED (12) which blinks to indicate a low battery condition.

In this example of the device of the present invention, the battery, fan, motor, and circuitry are designed to require very low power draw, enabling the device to run continuously for a long period of time. Preferably the device of this example continuously draws less than 20 mA, more preferably less than 10 mA, and most preferably less than 8 mA. To require infrequent battery replacement, the replaceable power supply of this example preferably is designed to last at least one month, more preferably at least two months, yet more preferably at least three months, most preferably at least 4 months.

To facilitate ease of use to the user of the device, an indicator is preferred to announce the approaching need to replace the battery. An indicator could be audible, such as a buzzer or whistle; or visual such as a blinking light or raised flag for example. In this example, the indicator is a blinking light emitting diode (LED) which blinks a few dozen times per minute when the voltage of the replaceable power unit falls below about 0.9 V. The LED of this example operates at 3.0 V, much higher than the voltage supplied by the replaceable battery. To enable the operation of this LED, a permanent 3.0 V lithium cell is mounted to the circuit board to power this circuit. The lithium cell is expected to last the lifetime of the device, approximately 5 years.

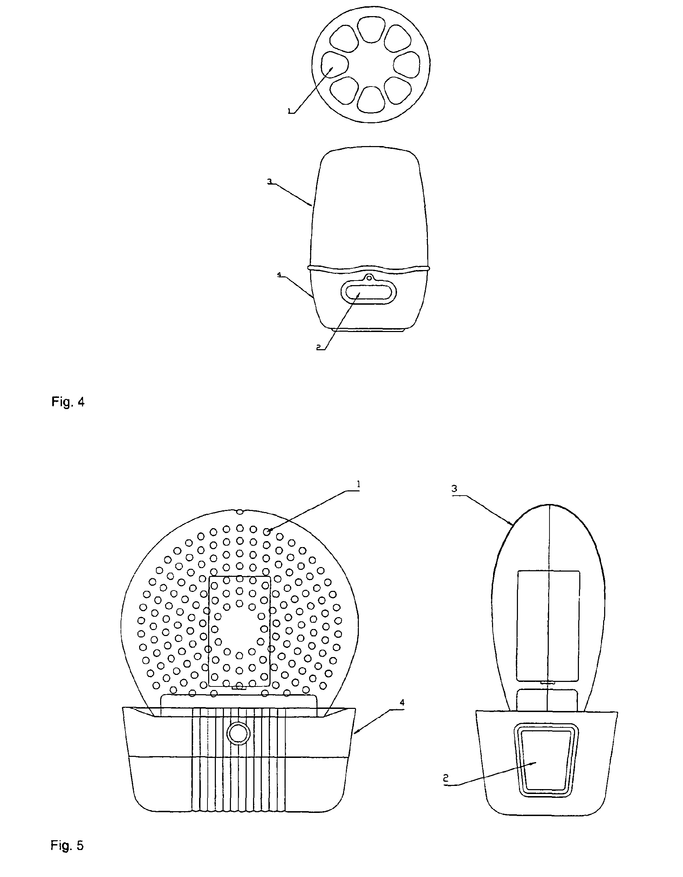

Fig. 4 is a schematic drawing of an alternate example of the device of the present invention. In Figure 4, a device is presented which comprises an air inlet (1), air outlets (2) on each side of the base (4) and a removable and replaceable filter element (3). The filter element is placed onto the base such that air flow from a fan draws air through the top of the filter element and through the air outlets on the base.

In this example, the filter member contains a filter medium and a battery. The filter medium is designed to exhibit the same useful lifetime as that of battery so that both may be replaced as a single unit. The filter member contains two metal contacts (not shown) allowing an electrical current to flow from the battery to the base. The filter medium of the device utilizes the same activated carbon and potassium permanganate system as described in the example of Fig. 1. The filter medium is shaped such that a 1.5 volt dry alkaline "D" battery fits inside a void in the filter.

Similar to the preferred mode of the device of the present invention, the base contains a fan, motor and a circuit board which controls the motor and an LED (not shown) which blinks to indicate the upcoming need to replace the filter member containing the battery and filter medium. Further, the base also contains metal contacts allowing electrical current to flow from the battery (contained in the filter assembly) to the motors.

Fig. 5 is a schematic drawing of an alternate example of the device of the present invention. In Figure 5, a device is presented which comprises an air inlet (2) on each side of the base (4), multiple air outlets (1), and a removable and replaceable filter element (3). The filter element is placed onto the base such that air flow from a fan draws air through the air inlets in the base, through the filter medium, and out through the air outlets in the filter member.

In this example, the filter member contains a filter medium and a battery. The filter medium is designed to exhibit the same useful lifetime as that of battery so that both may be replaced as a single unit. The filter member contains two metal contacts (not shown) allowing an electrical current to flow from the battery to the base. The filter medium of the device utilizes the same activated carbon and potassium permanganate system as described in the example of Fig 1. The filter medium is shaped such that air is forced from the base into the bottom and middle of the filter and then must travel outward through the filter medium to flow through the air outlets. Further, this filter is shaped such that a 1.5 volt dry alkaline "D" battery fits inside a void in the filter.

In this example, the base may contain multiple fans and motors to provide more air flow through the filter assembly. Because of the filter housing geometry, the best performance with this device is obtained by using two centrifugal fans, powered by one or two motors. The base contains a circuit board which controls the motors and an LED (not shown) which blinks to indicate the need to replace the filter member containing the battery and filter medium. Further, the base also contains metal contacts allowing current to flow from the battery (contained in the filter assembly) to the motors. Optionally, the base may be designed to contain the replaceable battery, eliminating the battery from the filter unit.

## Claims

1. Air filtering device for filtering air in confined spaces such as refrigerators, food storage closets, pantries, coolers and the like, comprising a first filtering member, a second filtering member, and an air flow conduit defining an air flow path through the device from an air inlet to an air outlet, said first filtering member and said second filtering member being positioned to interact with at least a portion of the molecules flowing along the air flow path or being adsorbed in one of the filter members,
**characterized in that**
said first filtering member is capable of removing malodor molecules from the air and comprises activated carbon supported on a support member, and said second filtering member is capable of oxidizing ethylene and comprises potassium permanganate supported on a zeolite or on alumina and wherein less than 30% of the macroscopic surface of the potassium permanganate is immediately exposed to air and at least 30% of the macroscopic surface of the potassium permanganate is in contact with activated carbon.

2. An air filtering device according to claim 1
wherein
said air filtering device further comprises an air ventilation member for moving air along the air flow path.

3. An alr filtering device according to claim 2
wherein
said air ventilation member moves at least 100 ml of air per second through said air inlet into said device.

4. An air filtering device according to claim 1
wherein
said support member comprises a foam, a woven, or a nonwoven web material.

5. An air filtering device according to claim 4
wherein
said activated carbon is supported on a polyurethane foam having a carbon density of between 0.01 and 0.3 grams per cm³.

6. An air filtering device according to claim 1
wherein
said first filter member comprises between 5 and 30 grams of activated carbon.

7. An air filtering device according to claim 1
wherein
said potassium permanganate is included as part of a solid particle supported in a foam structure or layered between non-woven or woven materials.

8. An air filtering device according to claim 1
wherein
at least 50% of the macroscopic surface of the potassium permanganate is in contact with activated carbon.

9. An air filtering device according to claim 1
wherein
said potassium permanganate is configured relative to said air flow path such that air flowing along said air flow path does not flow through the potassium permanganate.

## Patentansprüche

1. Luftfiltervorrichtung zum Filtern von Luft in abgeschlossenen Räumen wie Kühlschränken, Vorratschränken, Vorratsräumen, Kühlräumen und Ähnlichem, umfassend ein erstes Filterelement, ein zweites Filterelement und einen Luftkanal zur Definition eines Luftströmungswegs durch die Vorrichtung von einem Lufteinlass zu einem Luftauslass, wobei das erste Filterelement und das zweite Filterelement so angeordnet sind, dass sie zumindest mit einem Teil der Moleküle, die entlang des Luftströmungswegs strömen, oder die in einem der Filterelemente absorbiert sind, in Wechselwirkung treten,
**dadurch gekennzeichnet, dass**
das erste Filterelement in der Lage ist, schlecht riechende Moleküle aus der Luft zu entfernen, und Aktivkohle umfasst, die auf einem Trägerelement getragen wird, und das zweite Filterelement in der Lage ist, Ethylen zu oxidieren, und Kaliumpermanganat umfasst, das auf einem Zeolith oder auf Aluminiumoxid getragen wird, und worin weniger als 30 % der makroskopischen Oberfläche des Kaliumpermanganats unmittelbar der Luft ausgesetzt sind und mindestens 30 % der makroskopischen Oberfläche des Kaliumpermanganats sich in Kontakt mit der Aktivkohle befinden.

2. Luftfiltervorrichtung nach Anspruch 1, worin die Luftfiltervorrichtung ferner ein Luftventilationselement zur Bewegung der Luft entlang des Strömungswegs umfasst.

3. Luftfiltervorrichtung nach Anspruch 2, worin das Luftventilationselement mindestens 100 ml Luft pro Sekunde durch den Lufteinlass in die Vorrichtung bewegt.

4. Luftfiltervorrichtung nach Anspruch 1, worin das Trägerelement einen Schaumstoff, ein Gewebe oder ein Vliesmaterial umfasst.

5. Luftfiltervorrichtung nach Anspruch 4, worin die Aktivkohle auf einem Polyurethanschaum getragen wird und eine Kohlenstoffdichte zwischen 0,01 und 0,3 Gramm pro cm³ aufweist..

6. Luftfiltervorrichtung nach Anspruch 1, worin das erste Filterelement zwischen 5 und 30 Gramm Aktivkohle umfasst.

7. Luftfiltervorrichtung nach Anspruch 1, worin das Kaliumpermanganat als Teil fester Partikel in einer Schaumstoffstruktur getragen wird oder zwischen Vliesmaterialien geschichtet ist.

8. Luftfiltervorrichtung nach Anspruch 1, worin mindestens 50 % der makroskopischen Oberfläche des Kaliumpermanganats sich in Kontakt mit der Aktivkohle befinden.

9. Luftfiltervorrichtung nach Anspruch 1, worin das Kaliumpermanganat im Verhältnis zum Luftstrom so angeordnet ist, dass die entlang des Strömungsweges strömende Luft nicht durch das Kaliumpermanganat strömt.

## Revendications

1. Dispositif de filtrage d'air pour filtrer l'air dans des espaces confinés tels que des réfrigérateurs, des armoires à stockage d'aliments, des garde-manger, des chambres froides et similaires, comprenant un premier élément filtrant, un deuxième élément filtrant, et une conduite de débit d'air définissant une voie de débit d'air à travers le dispositif d'une entrée d'air à une sortie d'air, ledit premier élément filtrant et ledit deuxième élément filtrant étant positionnés de façon à interagir avec au moins une partie des molécules s'écoulant le long de la voie de débit d'air ou étant absorbée dans un des éléments filtrants, **caractérisé en ce que** ledit premier élément filtrant est susceptible d'éliminer les molécules de mauvaise odeur de l'air et comprend du carbone actif supporté sur un élément de support, et ledit deuxième élément filtrant est susceptible d'oxyder l'éthylène et comprend du permanganate de potassium supporté sur une zéolite ou sur de l'alumine et où moins de 30 % de la surface macroscopique du permanganate de potassium est immédiatement exposée à l'air et au moins 30 % de la surface macroscopique du permanganate de potassium est en contact avec du carbone actif.

2. Dispositif de filtrage d'air selon la revendication 1, où ledit dispositif de filtrage d'air comprend en outre un élément de ventilation d'air pour déplacer l'air le long de la voie de débit d'air.

3. Dispositif de filtrage d'air selon la revendication 2, dans lequel ledit élément de ventilation d'air déplace au moins 100 mL d'air par seconde à travers ladite entrée d'air dans ledit dispositif.

4. Dispositif de filtrage d'air selon la revendication 1, dans lequel ledit élément de support comprend un matériau en mousse, en nappe tissée ou non tissée.

5. Dispositif de filtrage d'air selon la revendication 4, dans lequel ledit carbone actif est supporté sur une mousse de polyuréthane ayant une masse volumique de carbone entre 0,01 et 0,3 grammes par cm³.

6. Dispositif de filtrage d'air selon la revendication 1, dans lequel ledit premier élément filtrant comprend entre 5 et 30 grammes de carbone actif.

7. Dispositif de filtrage d'air selon la revendication 1, dans lequel ledit permanganate de potassium est inclus en tant que partie de particule solide supportée dans une structure en mousse ou en couches entre des matériaux non-tissés.

8. Dispositif de filtrage d'air selon la revendication 1, dans lequel au moins 50 % de la surface macroscopique du permanganate de potassium est en contact avec du carbone actif.

9. Dispositif de filtrage d'air selon la revendication 1, dans lequel ledit permanganate de potassium est configuré par rapport à ladite voie de débit d'air de telle sorte que l'air s'écoulant le long de ladite voie de débit d'air ne s'écoule pas à travers le permanganate de potassium.
